# EUROPÄISCHE PATENTSCHRIFT

(11) **EP 1 818 069 B1**
(45) Veröffentlichungstag und Bekanntmachung des Hinweises auf die Patenterteilung: **03.09.2008**
(21) Anmeldenummer: 06002901.4
(22) Anmeldetag: 14.02.2006
(51) Int. Cl.: A61M 5/00, B65B 3/00

(54) **Verfahren zum Herstellen von vorfüllbaren Spritzen**
Process for manufacturing syringes to be pre-filled
Procédé de fabrication de seringues à pré-remplir

(43) Veröffentlichungstag der Anmeldung: 15.08.2007
(73) Patentinhaber: Gerresheimer Bünde GmbH, 32257 Bünde (DE)
(72) Erfinder: Wittland Frank, 32130 Enger (DE); Brandhorst Erik, 32120 Hiddenhausen (DE)
(74) Vertreter: Philipp, Matthias

(56) Entgegenhaltungen:
- US-A- 4 628 969
- US-A- 5 620 425
- US-B1- 6 263 641

## Beschreibung

Die Erfindung betrifft ein Verfahren zum Herstellen von vorfüllbaren Spritzen, mit den Schritten: a) Herstellen von Spritzenkörpern mit einem zylindrischen Trommelabschnitt, der ein erstes offenes Ende zur Aufnahme eines Spritzenkolbens und ein zweites offenes Ende zum Abgeben von Inhaltsstoffen aufweist, b) Reinigen einer inneren Oberfläche eines jeden Spritzenkörpers, und c) Silikonisieren durch Aufbringen einer Menge von Silikonöl oder -emulsion auf zumindest einen Teilbereich der inneren Oberfläche jedes Spritzenkörpers.

Ein derartiges Verfahren ist beispielsweise aus der US 6,263,641 B1 bekannt, bei der weiterhin vorgesehen ist, daß die Spritzenkörper nach dem Silikonisieren in einem Array angeordnet und in einem Behälter verpackt werden. Es kann sich um Spritzenkörper aus Kunststoff oder Glas handeln. Ein ähnliches Verfahren wird in der US-A-5 620 425 beschrieben.

Wenn solchermaßen hergestellte Spritzenkörper mit einem gewünschten injizierbaren Medikament gefüllt werden, kann es vorkommen, daß ein Teil des an sich medizinisch unbedenklichen und neutralen Silikonöls in unerwünschter Weise mit dem Medikament in Wechselwirkung tritt und dessen Wirkung verschlechtert. Wenn das aufgebrachte Silikonöl oder die Silikonemulsion im Zuge der Herstellung der Spritzen eingebrannt worden ist, d.h. einer Temperaturbehandlung von ca. 120°C bis zu 300°C und mehr unterworfen worden ist, liegt zwar ein Teil des Silikons kovalent oder nebenvalent an die Glasoberfläche der Spritze gebunden vor, es kann aber dennoch aufgrund z.B. bereichsweise übermäßigen Auftrags von Silikonöl ein unerwünschter Übergang in das Medikament auftreten.

Die Aufgabe der vorliegenden Erfindung besteht daher darin, eine Beeinträchtigung der Wirkung eines in eine vorfüllbare Spritze gefüllten Medikaments aufgrund einer Zusammenwirkung mit nicht ausreichend gebundenem Silikonöl weitestgehend zu verhindern.

Diese Aufgabe wird erfindungsgemäß bei einem gattungsgemäßen Verfahren durch die Schritte gelöst: d) Entfernen eines Teils der aufgebrachten Silikonmenge, e) Verpacken der Spritzenkörper, wobei die Schritte c) bis e) unter kontrollierten Reinraumbedingungen, insbesondere der ISO Klasse 8 und unter Arbeitsbedingungen entsprechend EN ISO 14644-1 mit laminarer Strömung (z.B. Klasse B), oder besser, ausgeführt werden. Unter "kontrollierten Reinraumbedingungen" wird im Rahmen der Erfindung eine ständige Überwachung der Luftqualität auf Einhaltung festgelegter Mindeststandards wie beispielsweise Partikelanzahl oder koloniebildende Einheiten je Kubikmeter verstanden.

Bevorzugt sieht die Erfindung vor, daß zwischen den Schritten c) und d) das aufgebrachte Silikonöl durch Wärmebehandlung bei Temperaturen zwischen 120°C und 350°C fixiert ("eingebrannt") wird. Hierbei kann weiter vorgesehen sein, daß nicht gebundenes oder nicht kovalent bzw. nebenvalent gebundenes Silikon ganz oder teilweise entfernt wird, beispielsweise 10% bis 90% der ursprünglich aufgebrachten Menge, oder 10% bis 100% des nicht kovalent bzw. nebenvalent gebundenen Anteils der ursprünglichen Menge.

Insbesondere kann vorgesehen sein, daß in Schritt d) mit Wasser oder einem Lösungsmittel wie etwa Alkohol gespült wird. Dies kann bei einer Temperatur im Bereich von 20°C bis 100°C, insbesondere 75°C bis 95°C und bevorzugt bei 85°, erfolgen. Es ist vorteilhaft, wenn nach dem Spülen mit sterilisierter Luft getrocknet wird.

Weiterhin kann vorgesehen sein, daß vor Schritt e) ein Nadelschutz oder ein Verschluß (Tip Cap) aufgesetzt wird.

In Schritt e) kann eine Anzahl von Spritzenkörpern in ein Tray eingelegt, das Tray in ein Behältnis eingesetzt dieses mit einer Abdeckung verschlossen und versiegelt und das Behältnis verpackt werden. Anschließend kann vorgesehen sein, daß das verpackte Behältnis sterilisiert wird.

In einer Variante kann vorgesehen sein, daß in Schritt a) hergestellte Spritzenkörper verpackt und vor Schritt b) unter kontrollierten Reinraumbedingungen entpackt werden. Hierbei ist es zweckmäßigerweise so, daß die Spritzenkörper unter Reinraumbedingungen der ISO Klasse 8 und unter Arbeitsbedingungen entsprechend EN ISO 14644-1 mit laminarer Strömung, oder besser, ver- und entpackt werden.

Die Erfindung sieht weiter vor, daß Schritt e) unter kontrollierten Reinraumbedingungen der ISO Klasse 8 und unter Arbeitsbedingungen entsprechend EN ISO 14644-1 mit laminarer Strömung, oder besser, durchgeführt wird.

Sofern die Silikonisierung eingebrannt wird, kann vorgesehen sein, daß ein gekapselter Ofen verwendet wird, der an einen Reinraumbereich, z.B. der ISO Klasse 8 oder besser, angeschlossen ist.

In einer weiteren Variante der Erfindung kann vorgesehen sein, daß in Schritt c) hergestellte Spritzenkörper verpackt und vor Schritt d) entpackt werden. Auch hier besteht die Möglichkeit, daß die Spritzenkörper unter kontrollierten Reinraumbedingungen, insbesondere der ISO Klasse 8 und unter Arbeitsbedingungen entsprechend EN ISO 14644-1 mit laminarer Strömung, oder besser, ver- und entpackt werden.

In einer bevorzugten Ausgestaltung der Erfindung ist vorgesehen, daß die Schritte b) bis e) unter kontrollierten Reinraumbedingungen, insbesondere der ISO Klasse 8 und unter Arbeitsbedingungen entsprechend EN ISO 14644-1 mit laminarer Strömung, oder besser, durchgeführt werden.

Weitere Vorteile und Merkmale der Erfindung ergeben sich aus der nachfolgenden Beschreibung eines bevorzugten Ausführungsbeispiels, wobei zur Erläuterung auf eine Zeichnung Bezug genommen ist, deren

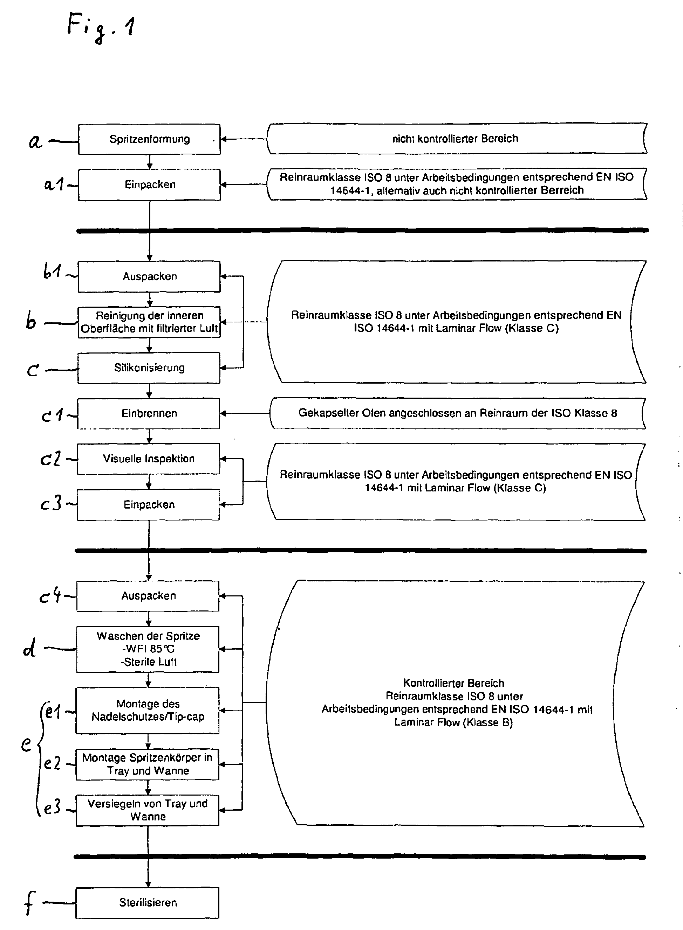

Fig. 1 beispielhaft einen möglichen Ablauf des erfindungsgemäßen Verfahrens nach Art eines Fließschemas zeigt.

Der beispielhafte Verfahrensablauf nach Fig. 1 erläutert die Herstellung von vorfüllbaren Spritzen bis hin zu deren Verpackung in Form eines Arrays von Spritzen in einem geschlossenen und sterilisierten Behältnis ("Tub").

Schritt a betrifft die Herstellung eines Spritzenkörpers als solchen unmittelbar durch Formung aus Kunststoff oder Glas mittels einer geeigneten Vorrichtung, die als solche bekannt ist und nicht näher beschrieben wird. In diesem Schritt werden Spritzenkörper mit einem zylindrischen Trommelabschnitt hergestellt, der ein erstes offenes Ende zur Aufnahme eines Spritzenkolbens sowie ein gegenüberliegendes, zweites offenes Ende mit kleinerem Querschnitt zur Abgabe von Inhaltsstoffen aufweist. An dem zweiten offenen Ende wird zu einem geeigneten Zeitpunkt des hier beschriebenen Verfahrens oder auch erst unmittelbar vor Verwendung der zu füllenden oder bereits gefüllten Spritze eine Injektionskanüle befestigt. Hierfür sind unterschiedliche Techniken in Gebrauch, beispielsweise Einkleben oder Aufstecken bzw. Aufrasten, die an sich bekannt sind und nicht näher beschrieben werden.

In Schritt a1 werden die derart hergestellten Spritzenkörper eingepackt oder verpackt, wobei dieser Verpackungsvorgang unter kontrollierten Reinraumbedingungen, hier der ISO Klasse 8 und unter Arbeitsbedingungen gemäß EN ISO 14644-1, durchgeführt werden kann. Alternativ erfolgen sowohl die Herstellung der Spritzenkörper als auch deren Verpackung in einem hinsichtlich des Reinheitsgrads nicht kontrollierten Produktionsbereich.

Schritt b1 bezeichnet das Auspacken der zuvor wie beschrieben verpackten Spritzenkörper unter kontrollierten Reinraumbedingungen, hier der ISO Klasse 8 und unter Arbeitsbedingungen gemäß EN ISO 14644-1 mit laminarer Strömung (z.B. Klasse C).

In einem weiteren Schritt b wird die innere Oberfläche des Spritzenkörpers gereinigt, beispielsweise mit gereinigter oder steriler Luft, oder auch mit ionisierter Luft, ebenfalls unter kontrollierten Bedingungen wie in Schritt b1.

Schritt c bezeichnet die Silikonisierung der inneren Oberfläche des Spritzenkörpers unter kontrollierten Bedingungen wie in Schritt b1 und b, wobei in geeigneter Weise Silikonöl oder eine Silikonemulsion auf die innere Oberfläche aufgebracht wird, beispielsweise durch Aufsprühen. Es kann hierbei eine vorbestimmte Menge an Silikon aufgebracht werden. Zusätzlich kann vorgesehen sein, überschüssiges Silikon sofort zu entfernen, beispielsweise durch Abwischen oder Spülen.

Gemäß Schritt c1 ist bevorzugt vorgesehen, daß das aufgebrachte Silikonöl in geeigneter Weise fixiert wird, in diesem Beispiel durch eine Temperaturbehandlung bei mindestens ca. 120°C (Autoklavieren) bis hin zu 350°C (Einbrennen) während einer vorgeschriebenen Behandlungsdauer. Für diese Temperaturbehandlung wird ein gekapselter Ofen verwendet, der mit einem Reinraumbereich, insbesondere mit einem Bereich der ISO Reinheitsklasse 8, verbunden ist.

Schritt c2 bezieht sich auf eine visuelle Inspektion der Spritzenkörper unter Reinraum- und Arbeitsbedingungen, insbesondere wie in den Schritten b1 bis c, und Schritt c3 bezeichnet eine Verpackung der Spritzenkörper unter entsprechenden Bedingungen.

Schritt c4 bezieht sich auf das Auspacken der in Schritt c3 verpackten Spritzenkörper, die anschließend in Schritt d in der vorgeschriebenen Weise mit WFI (Water for Injection, Spülwasser mit vorgeschriebener Reinheitsspezifikation) gespült werden, beispielsweise bei einer Temperatur von 85°C. Anschließend erfolgt eine Trocknung mit steriler Luft.

In Schritt e1 werden die Spritzenkörper in Abhängigkeit von der Art der jeweils zu fertigenden Spritzen mit weiteren Teilen versehen, beispielsweise mit einem Nadelschutz, einer Verschlußkappe (Tip Cap) und/oder weiteren Teilen.

Schritt e2 bezieht sich auf die Anordnung einer Gruppe von Spritzenkörpern in einem Tray, welches in ein wannenförmiges Behältnis ("Tub") eingelegt wird, das seinerseits nach Aufbringen einer Abdeckung verschlossen wird (Schritt e3).

Schritt f bezeichnet die Sterilisierung der in der Verpackung befindlichen Spritzenkörper und der Verpackung selbst, insbesondere mit Gas wie etwa ETO (Ethylenoxid). Dieser Schritt kann räumlich entfernt bzw. unabhängig von den vorangehenden Schritten durchgeführt werden.

Sämtliche Verfahrensschritte c4 bis e3 laufen in dem dargestellten Beispiel unter kontrollierten Reinraumbedingungen ab, hierbei unter Bedingungen der ISO Klasse 8 und unter Arbeitsbedingungen gemäß EN ISO 14644 mit laminarer Strömung (z.B. Klasse B), oder besser.

Wie in Fig. 1 mit horizontalen Trennbalken angedeutet ist, kann der ansonsten kontinuierliche Verfahrensablauf zwischen den Schritten a1 und b1 und/oder c3 und c4 und/oder e3 und f unterbrochen sein, indem die jeweils verpackten Spritzenkörper beispielsweise zwischengelagert werden, um dann bei Bedarf dem jeweiligen Entpackungsschritt b1 oder c4 oder Sterilisierungsschritt f zugeführt zu werden. Alternativ können die Ver- und Entpackungsschritte weggelassen und das Herstellverfahren vollständig kontinuierlich und durchgehend unter Reinraumbedingungen durchgeführt werden.

## Patentansprüche

1. Verfahren zum Herstellen von vorfüllbaren Spritzen, mit den Schritten:
a) Herstellen von Spritzenkörpern mit einem zylindrischen Trommelabschnitt, der ein erstes offenes Ende zur Aufnahme eines Spritzenkolbens und ein zweiten offenes Ende zum Abgeben von Inhaltsstoffen aufweist,
b) Reinigen einer inneren Oberfläche eines jeden Spritzenkörpers,
c) Silikonisieren durch Aufbringen einer Menge von Silikonöl oder Silikonemulsion auf zumindest einem Teilbereich der inneren Oberfläche jedes Spritzenkörpers,
c1) Fixieren der aufgebrachten Silikonmenge durch Wärmebehandlung bei Temperaturen zwischen 120° C und 350° C,
d) Entfernen zumindest eines Teils des nicht kovalent oder nebenvalent gebundenen Silikons,
e) Verpacken der Spritzenkörper, wobei die Schritte c) bis e) unter kontrollierten Reinraumbedingungen ausgeführt werden.

2. Verfahren nach Anspruch 1, **dadurch gekennzeichnet, daß** die Schritte c) bis e) durchgehend unter Reinraumbedingungen der ISO Klasse 8 und unter Arbeitsbedingungen entsprechend EN ISO 14644-1 mit laminarer Strömung, oder besser, ausgeführt werden.

3. Verfahren nach einem der vorangehenden Ansprüche, **dadurch gekennzeichnet, daß** in Schritt d) mit Wasser oder einem Lösungsmittel, etwa Alkohol, gespült wird.

4. Verfahren nach Anspruch 3, **dadurch gekennzeichnet, daß** bei einer Temperatur im Bereich von 20°C bis 120°C, etwa 75°C bis 95°C, gespült wird.

5. Verfahren nach Anspruch 3 oder 4, **dadurch gekennzeichnet, daß** nach dem Spülen mit steriler Luft getrocknet wird.

6. Verfahren nach einem der vorangehenden Ansprüche, **dadurch gekennzeichnet, daß** vor Schritt e) ein Nadelschutz oder ein Verschluß, etwa Tip Cap, aufgesetzt wird (d1).

7. Verfahren nach einem der vorangehenden Ansprüche, **dadurch gekennzeichnet, daß** in Schritt e) eine Anzahl von Spritzenkörpern in ein Tray eingelegt, das Tray in ein Behältnis eingesetzt, dieses mit einer Abdeckung verschlossen (e2) und versiegelt (e3) und das Behältnis verpackt wird.

8. Verfahren nach einem der vorangehenden Ansprüche, **dadurch gekennzeichnet, daß** in Schritt a) hergestellte Spritzenkörper verpackt (a1) und vor Schritt b) unter kontrollierten Reinraumbedingungen entpackt werden (b1).

9. Verfahren nach Anspruch 8, **dadurch gekennzeichnet, daß** die Spritzenkörper unter Reinraumbedingungen der ISO Klasse 8 und unter Arbeitsbedingungen entsprechend EN ISO 14644-1 mit laminarer Strömung, oder besser, ver- und entpackt werden.

10. Verfahren nach einem der vorangehenden Ansprüche, **dadurch gekennzeichnet, daß** Schritt e) unter Reinraumbedingungen der ISO Klasse 8 und unter Arbeitsbedingungen entsprechend EN ISO 14644-1 mit laminarer Strömung, oder besser, durchgeführt wird.

11. Verfahren nach Anspruch 1, **dadurch gekennzeichnet, daß** ein gekapselter Ofen verwendet wird, der an einen Reinraumbereich, z.B. der ISO Klasse 8 oder besser, angeschlossen ist.

12. Verfahren nach einem der vorangehenden Ansprüche, **dadurch gekennzeichnet, daß** in Schritt c) hergestellte Spritzenkörper verpackt (c3) und vor Schritt d) entpackt werden (c4).

13. Verfahren nach Anspruch 12, **dadurch gekennzeichnet, daß** die Spritzenkörper unter kontrollierten Reinraumbedingungen ver- und entpackt werden.

14. Verfahren nach Anspruch 13, **dadurch gekennzeichnet, daß** die Spritzenkörper unter kontrollierten Reinraumbedingungen der ISO Klasse 8 und unter Arbeitsbedingungen entsprechend EN ISO 14644-1 mit laminarer Strömung, oder besser, ver- und entpackt werden.

15. Verfahren nach einem der vorangehenden Ansprüche, **dadurch gekennzeichnet, daß** die Schritte b) bis e) unter kontrollierten Reinraumbedingungen durchgeführt werden.

16. Verfahren nach Anspruch 15, **dadurch gekennzeichnet, daß** die Schritte b) bis e) unter Reinraumbedingungen der ISO Klasse 8 und unter Arbeitsbedingungen entsprechend EN ISO 14644-1 mit laminarer Strömung, oder besser, durchgeführt werden.

## Claims

1. A process of producing pre-fillable syringes, comprising the following process stages:
a) producing syringe bodies having a cylindrical drum which comprises a first open end for receiving a syringe piston and a second open end for discharging contents materials;
b) cleaning an inner surface of each syringe body;
c) siliconizing by applying a quantity of silicone oil or silicone emulsion to at least a partial region of the inner surface of each syringe body;
c1) fixing the applied quantity of silicone by heat treatment at temperatures ranging between 120°C and 350°C;
d) removing at least part of the silicone bonded by covalency or secondary valency.
e) packing the syringe bodies, wherein the process stages c) to e) are carried out under controlled clean room conditions.

2. A process according to claim 1,
**characterised in**
**that** the process stages c) to e) are carried out continuously under clean room conditions of ISO Class 8 and under working conditions in accordance with EN ISO 14644-1 with laminar flow conditions or better.

3. A process according to any one of the preceding claims,
**characterised in**
**that** during the process stage d), rinsing takes place with water or a solvent, for example alcohol.

4. A process according to claim 3,
**characterised in**
**that** rinsing takes place at a temperature ranging between 20°C and 120°C, approximately 75°C and 95°C.

5. A process according to claim 3 or 4,
**characterised in**
**that** after the rinsing operation, drying takes place with sterile air.

6. A process according to any one of the preceding claims,
**characterised in**
**that** prior to process stage e), there is applied a needle protection or a seal, for example a tip cap (d1).

7. A process according to any one of the preceding claims,
**characterised in**
**that**, in process stage e), a number of syringe bodies is placed into a tray, that the tray is placed into a container which is closed (e2) and sealed (e3) by a cover and that the container is packed.

8. A process according to any one of the preceding claims,
**characterised in**
**that** the syringe bodies produced in stage a) are packed and, prior to stage b), unpacked under controlled clean room conditions (b1).

9. A process according to claim 8,
**characterised in**
**that** the syringe bodies are packed and unpacked under clean room conditions of ISO Class 8 and under working conditions according to EN ISO 14644-1 with laminar flow or better.

10. A process according to any one of the preceding claims,
**characterised in**
**that** process stage e) is carried out under clean room conditions of ISO Class 8 and under working conditions according to EN ISO 14644-1 with laminar flow or better.

11. A process according to claim 1,
**characterised in**
**that** use is made of an encapsulated oven which is connected to a clean room region, e.g. of ISO Class 8 or better.

12. A process according to any one of the preceding claims,
**characterised in**
**that** the syringe bodies produced in process stage c) are paced (c3e) and, prior to process stage d), unpacked (c4).

13. A process according to claim 12,
**characterised in**
**that** the syringe bodies are packed and unpacked under controlled clean room conditions.

14. A process according to claim 13,
**characterised in**
**that** the syringe bodies are packed and unpacked under controlled clean room conditions of ISO Class 8 and under working conditions according to EN ISO 14644-1 with laminar flow conditions or better.

15. A process according to any one of the preceding claims,
**characterised in**
**that** process stages b) to e) are carried out under controlled clean room conditions.

16. A process according to claim 15,
**characterised in**
**that** process stages b) to e) are carried out under clean room conditions of ISO Class 8 and under working conditions according to EN ISO 14644-1 with laminar flow conditions or better.

## Revendications

1. Procédé de fabrication de seringues à pré-remplir, comprenant les étapes de :
a) fabrication de corps de seringue ayant un tronçon de réservoir cylindrique, présentant une première extrémité ouverte, pour recevoir un piston de seringue, et une deuxième extrémité ouverte, pour délivrer des substances de contenu,
b) nettoyage d'une surface intérieure de chaque corps de seringue,
c) siliconage, par application d'une quantité d'huile de silicone, ou d'émulsion de silicone, sur au moins une zone partielle de la surface intérieure de chaque corps de seringue,
c1) fixation de la quantité de silicone appliquée, par traitement thermique à des températures comprises entre 120°C et 350°C,
d) enlèvement d'au moins une partie du silicone, non lié de façon covalente ou par valence auxiliaire,
e) emballage du corps de seringue, sachant que les étapes c) à e) sont exécutées dans des conditions de salle propre contrôlées.

2. Procédé selon la revendication 1, **caractérisé en ce que** les étapes c) à e) sont exécutées en continu, dans des conditions de salle propre de la classe 8 de l'ISO, et dans des conditions de travail correspondant à EN ISO 14644-1 avec écoulement laminaire, ou mieux.

3. Procédé selon l'une des revendications précédentes, **caractérisé en ce que**, à l'étape d), on rince à l'eau ou avec un solvant, par exemple de l'alcool.

4. Procédé selon la revendication 3, **caractérisé en ce que** l'on effectue le rinçage à une température dans la fourchette de 20°C à 120°C, par exemple de 75°C à 95°C.

5. Procédé selon la revendication 3 ou 4, **caractérisé en ce que** l'on pratique un séchage à l'air stérile après le rinçage.

6. Procédé selon l'une des revendications précédentes, **caractérisé en ce que**, avant l'étape e), on pose une protection d'aiguille ou une fermeture, par exemple de type Tip Cap (d1).

7. Procédé selon l'une des revendications précédentes, **caractérisé en ce que**, à l'étape (e), on insère une pluralité de corps de seringue dans un plateau, on introduit le plateau dans un récipient, on ferme celui-ci avec un couvercle (d2) et l'on scelle (e3), et l'on procède à l'emballage du récipient.

8. Procédé selon l'une des revendications précédentes, **caractérisé en ce que** des corps de seringue, ayant été fabriqués à l'étape a), sont emballés (a1) et déballés avant l'étape b), dans des conditions de salle propre contrôlées (b1) .

9. Procédé selon la revendication 8, **caractérisé en ce que** les corps de seringue sont emballés et déballés dans des conditions de salle propre de la classe 8 de l'ISO, et dans des conditions de travail correspondant à EN ISO 14644-1 avec écoulement laminaire, ou mieux.

10. Procédé selon l'une des revendications précédentes, **caractérisé en ce que** l'étape e) est exécutée dans des conditions de salle propre de la classe 8 de l'ISO, et dans des conditions de travail correspondant à EN ISO 14644-1 avec écoulement laminaire, ou mieux.

11. Procédé selon la revendication 1, **caractérisé en ce que** l'on utilise un four encapsulé, raccordé à une zone de salle propre, par exemple de la classe 8 de l'ISO, ou mieux.

12. Procédé selon l'une des revendications précédentes, **caractérisé en ce que** des corps de seringue sont emballés et déballés dans des conditions de salle propre.

13. Procédé selon la revendication 12, **caractérisé en ce que** les corps de seringue, ayant été fabriqués à l'étape a), sont emballés (a1) et déballés avant l'étape b), dans des conditions de salle propre contrôlées (b1).

14. Procédé selon la revendication 13, **caractérisé en ce que** les corps de seringue sont emballés et déballés dans des conditions de salle propre contrôlées de la classe 8 de l'ISO, et dans des conditions de travail correspondant à EN ISO 14644-1 avec écoulement laminaire, ou mieux.

15. Procédé selon l'une des revendications précédentes, **caractérisé en ce que** les étapes c) à e) sont exécutées dans des conditions de salle propre contrôlées.

16. Procédé selon la revendication 15, **caractérisé en ce que** les étapes c) à e) sont exécutées dans des conditions de salle propre de la classe 8 de l'ISO, et dans des conditions de travail correspondant à EN ISO 14644-1 avec écoulement laminaire, ou mieux.
